# FASCICULE DE BREVET EUROPEEN

(11) **EP 0 759 292 B1**
(45) Date de publication et mention de la délivrance du brevet: **26.03.1997**
(21) Numéro de dépôt: 96400653.0
(22) Date de dépôt: 27.03.1996
(51) Int. Cl.: A61K 7/06

(54) **Utilisation de derivés de l'hormone stimulatrice des melanocytes de type alpha pour stimuler la pousse des cheveux**
Verwendung von Derivaten des Melanocyte-stimulierenden Hormons vom Typ alpha zur Stimulierung des Haarwachstums
Use of alpha type melanocytes stimulating hormone derivatives to stimulate the hairgrowth

(30) Priorité: 28.04.1995 FR 9505158
(43) Date de publication de la demande: 26.02.1997
(73) Titulaire: L'OREAL, 75008 Paris (FR)
(72) Inventeur: Mahe, Yann, 91390 Morsang-sur-Orge (FR)
(74) Mandataire: Tezier Herman, Béatrice

(56) Documents cités:
- EP-A- 0 293 837
- WO-A-88/08695
- WO-A-91/07431
- PATENT ABSTRACTS OF JAPAN vol. 13, no. 515 (C-655) [3863] & JP-A-01 207225 (TAIYO KAGAKU CO)
- STN, Serveur de bases de données, XP002006226
- STN, Serveur de bases de données, XP002006226, Karlsruhe, DE, fichier Chemical Abstracts, vol. 118, nA 248073

## Description

La présente invention concerne l'utilisation à titre de principe actif, dans un milieu physiologiquement acceptable, dans une composition cosmétique ou pour la préparation d'un médicament, d'une quantité efficace d'au moins un peptide contenant le tripeptide Lysine-Proline-Valine, à l'exception de tout peptide ne contenant que la séquence Lysine-Proline-Valine immédiatement précédée d'une Histidine, ou de tout équivalent biologique fonctionnel, destinée à induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.
A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.
A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.
Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.
Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

On recherche depuis de nombreuses années, dans l'industrie cosmétique ou pharmaceutique, des substances permettant de supprimer ou de réduire l'effet de l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-piperidinopyrimidine 3-oxyde ou "Minoxidil" décrit dans US 4 596 812 ou encore ses nombreux dérivés comme ceux décrits par exemple dans les demandes de brevet EP 353123, EP 356271, EP 408442, EP 522964, EP 420707, EP 459890, EP 519819.
On peut encore citer le 6-amino 1,2-dihydro 1-hydroxy 2-imino 4-pipéridino pyrimidine et ses dérivés, qui sont décrits plus particulièrement dans le brevet US-A-4 139 619.

Il reste, d'une manière générale, qu'il serait intéressant et utile de pouvoir disposer de composés actifs autres que ceux déjà connus, potentiellement plus actifs et/ou moins toxiques. Un des axes envisageable est la recherche de composés agissant soit sur le follicule pileux en favorisant sa pousse soit sur le cycle pilaire en allongeant par exemple la phase anagène, ce qui aurait pour effet de retarder le passage dans les phases de repos et donc de ralentir la fréquence des cycles.

Or, la demanderesse vient maintenant de découvrir, après d'importantes recherches menées sur la question, qu'un peptide contenant le tripeptide Lysine-Proline-Valine, ou tout équivalent biologique fonctionnel à l'exception de tout peptide ne contenant que la séquence Lysine-Proline-Valine immédiatement précédée d'une Histidine, permet d'induire et/ou de stimuler la croissance des cheveux, et/ou de diminuer leur chute, de manière efficace.
Par équivalent biologique fonctionnel, on entend un peptide fonctionnellement équivalent en terme de fonction biologique dont l'un au moins des résidus d'acide aminé peut avoir été changé pour un résidu d'acide aminé ayant un index hydropathique similaire.
L'index hydropathique est un index attribué aux acides aminés en fonction de leur hydrophobicité et de leur charge (Kyte et al. (1982), J. Mol. Biol., 157 : 105).
Cette découverte est à la base de la présente invention.

Lorsque l'on utilise un peptide contenant le tripeptide Lysine-Proline-Valine, il est bien entendu que celui-ci est choisi de telle sorte que les acides aminés entourant le motif Lysine-Proline-Valine, tant par leur nature que par la structure secondaire du peptide qu'ils vont induire, n'empêchent pas celui-ci d'exercer l'activité pour laquelle il est utilisé dans la présente invention.

Le tripeptide Lysine-Proline-Valine se retrouve par exemple dans la séquence peptidique de l'hormone stimulatrice des mélanocytes de type α (α-MSH) ou Mélanotropine.
L'α-MSH fut décrite à l'origine comme produite par la glande pituitaire, mais le cerveau en général, le sang, la peau et d'autres tissus sont capables de produire une activité α-MSH.
Dans l'épiderme, il a été montré par Schauer et coll. (J. Clin. Invest. 93, May 1994 pp 2258-2262) que les kératinocytes sont une source d'α-MSH.
Des récepteurs à l'α-MSH sont présents dans les follicules de scalp humain (Pigment cell Res. 4:193-8, 1991).

L'α-MSH (1-13) est connue pour son activité antipyrétique, son activité anti-inflammatoire et son activité propigmentante.
Le signal antipyrétique de l'α-MSH réside dans sa séquence carboxy-terminale et peut-être mimé par le tripeptide 11-13 carboxy-terminal (L)Lys(L)Pro(L)Val (Watanabe et al. Brain research Bulletin, Vol. 32, pp 311-314, 1993).
Ce neuropeptide est connu pour inhiber l'inflammation induite par des cytokines ou d'autres médiateurs de l'inflammation ainsi que par des irritants. Ainsi, les brevets US 5028592 et WO 88/00833 visent à protéger l'utilisation du tripeptide (L ou D)Lys-(L)Pro-(L ou D)Val dans un procédé de traitement anti-inflammatoire et dans la préparation d'un médicament pour traiter l'inflammation.
L'activité propigmentante de l'αMSH est décrite dans la demande de brevet européen EP-A-0293837 ou encore dans la demande de brevet japonais JP-A-01207225.

Ainsi, l'invention concerne l'utilisation, dans une composition cosmétique ou pour la préparation d'un médicament, d'une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine,, destinée à induire et/ou stimuler la croissance des cheveux et/ou freiner leur chute

Plus particulièrement, l'invention concerne l'utilisation dans une composition cosmétique ou pour la préparation d'un médicament d'une quantité efficace du tripeptide Lysine-Proline-Valine, destinée à induire et/ou stimuler la croissance des cheveux et/ou freiner leur chute.

Dans le texte qui va suivre, de manière générale, le terme "peptide" couvre aussi bien le "peptide contenant le tripeptide Lysine-Proline-Valine, ou tout équivalent biologique fonctionnel à l'exception de tout peptide ne contenant que la séquence Lysine-Proline-Valine immédiatement précédée d'une Histidine, ", que le "tripeptide Lysine-Proline-Valine" isolé.
Cependant dans les cas où l'utilisation de termes précis s'avère impérative, les termes "peptide contenant le tripeptide Lysine-Proline-Valine, ou tout équivalent biologique fonctionnel à l'exception de tout peptide ne contenant que la séquence Lysine-Proline-Valine immédiatement précédée d'une Histidine, " et "tripeptide Lysine-Proline-Valine" seront utilisés sous cette forme afin de ne désigner que l'une ou l'autre forme de l'objet utilisé dans l'invention.

Le peptide utilisé selon l'invention peut bien entendu être d'origine naturelle. Cela sous-entend qu'il peut avoir été purifié à partir de matériel biologique naturel. On peut à cet égard citer en exemple l'α-MSH, largement présente dans le système nerveux central et qu'il est entre autre possible de purifier à partir de glande pituitaire.
Cependant, avec les progrès du génie chimique, il est maintenant aisé de synthétiser à façon des peptides, même de longueur importante.

Ainsi, l'invention a plus précisément pour objet l'utilisation d'une quantité suffisante du peptide tel que défini précédemment, caractérisée par le fait que le peptide est un peptide d'origine naturelle ou synthétique.

Dans le domaine des acides aminés, la géométrie des molécules est telle qu'elles peuvent théoriquement se présenter sous la forme d'isomères optiques différents. Il existe en effet une conformation moléculaire de l'acide aminé (aa) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa).

La nature n'a retenu pour les acides aminés naturels que la conformation lévogyre. En conséquence si le peptide utilisé dans les compositions selon l'invention est d'origine naturelle, celui-ci sera constitué d'acides aminés de type L-aa.
Cependant, la synthèse chimique en laboratoire permet de préparer des d'acides aminés ayant les deux conformations possibles. A partir de ce matériel de base il est possible d'incorporer lors de la synthèse de peptides aussi bien des d'acides aminés sous la forme d'isomères optiques dextrogyre ou lévogyre.
On peut ainsi incorporer lors de la synthèse de peptides des résidus d'acides aminés Lysine, Proline, Valine indifféremment sous leur forme D-Lysine (D-Lys), L-Lysine (L-Lys), D-Proline (D-Pro), L-Proline (L-Pro), D-Valine (D-Val) ou L-Valine (L-Val).

Ainsi, l'invention concerne plus particulièrement l'utilisation d'une quantité suffisante du peptide tel que défini précédemment, caractérisée par le fait que les résidus acides aminés du tripeptide Lysine-Proline-Valine constituant le peptide sont indifféremment sous la forme d'isomères optiques dextrogyre ou lévogyre.

On peut citer ainsi les peptides contenant au moins l'un des tripeptides suivant :
D-Lys-D-Pro-D-Val,
D-Lys-D-Pro-L-Val,
D-Lys-L-Pro-D-Val,
L-Lys-D-Pro-D-Val,
D-Lys-L-Pro-L-Val,
L-Lys-D-Pro-L-Val,
L-Lys-L-Pro-D-Val,
L-Lys-L-Pro-L-Val.

Selon l'invention, il peut bien entendu être utilisé plus d'un peptide. Dans ce cas, le mélange de peptides peut être constitué par l'une des combinaisons possibles des peptides ci-dessus décrits.

Il se peut que pour des questions de résistance à la dégradation il soit nécessaire d'utiliser selon l'invention une forme protégée du peptide. La forme de la protection doit évidement être une forme biologiquement compatible. De nombreuses formes de protections biologiquement compatibles peuvent être envisagées comme par exemple l'acylation ou l'acétylation de l'extrémité amino-terminale ou l'amidation de l'extrémité carboxy-terminale.

Ainsi, l'invention concerne une utilisation telle que précédemment définie, caractérisée par le fait que le peptide est sous une forme protégée ou non.

De préférence, on utilise selon l'invention une protection basée soit sur l'acylation ou l'acétylation de l'extrémité amino-terminale, soit sur l'amidation de l'extrémité carboxy-terminale soit encore sur les deux.

La quantité efficace d'actif correspond à la quantité nécessaire pour obtenir le résultat désiré.

Plus particulièrement, dans la composition cosmétique, le peptide est présent dans une quantité telle que le tripeptide Lysine-Proline-Valine est à une concentration comprise entre 10⁻¹² M et 10⁻³ M et de préférence comprise entre 10⁻⁹ M et 10⁻⁴ M.
Plus particulièrement, dans la préparation d'un médicament, le peptide est présent dans une quantité telle que le tripeptide Lysine-Proline-Valine peut être utilisé à une concentration comprise entre 10⁻¹² M et 1 M et de préférence comprise entre 10⁻⁶ M et 10⁻¹ M.

Il est clair que l'homme du métier sait ajuster cette quantité de matériel selon qu'il utilise le peptide contenant le tripeptide Lysine-Proline-Valine, ou tout équivalent biologique fonctionnel ou le tripeptide Lysine-Proline-Valine.

Le médicament selon l'invention peut être administré par voie parentérale, entérale ou encore par voie topique. De préférence, le médicament est administré par voie topique.

Dans la demande de brevet n°95-01881 déposée en France, la demanderesse a décrit une phase inflammatoire de l'alopécie.
Le terme alopécie recouvre toute une famille d'atteintes du follicule pileux ayant pour conséquence, quelqu'en soit la raison, la perte définitive partielle ou générale des cheveux.
On peut citer par exemple l'alopécie androgénétique, l'alopecia areata (la pelade), ou l'alopecia totalis, ou encore l'alopecia universalis.

Sans vouloir se lier à une quelconque théorie, il semble que certaines alopécies passent par au moins une phase inflammatoire

La phase inflammatoire de l'alopécie se caractérise, entre autres, par une modification du niveau d'expression de médiateurs de l'inflammation.
Parmi ces médiateurs, on peut citer les cytokines, dont en particulier l'interleukine-1α, l'interleukine-1β, l'interleukine-6, les facteurs de nécrose tumorale-α et β (TNF-α et β), les chémokines comme l'interleukine-8 ou le facteur chimiotactique et activateur des monocytes (MCAF), ou encore d'autres facteurs chimiotactiques responsables du recrutement des cellules lymphocytaires, monocytaires, de Langerhans ou basophiles au niveau du site inflammatoire, tels que les leukotriènes B₄, ou encore d'autres facteurs impliqués dans la cascade inflammatoire, tels que l'acide arachidonique, ou les prostaglandines, dont en particulier les prostaglandines E₂.

La demanderesse a trouvé que chez certains sujets, et en particulier chez ceux présentant un début d'alopécie, le taux des interleukines est modifié. Le taux d'interleukine-1α et d'interleukine-8, et plus particulièrement le taux d'interleukine-1α, est augmenté chez la plupart des sujets présentant un début d'alopécie.
La demanderesse a également trouvé que chez certains sujets présentant une alopécie avancée le taux des prostaglandines E₂ est plus élevé que chez d'autres, suggérant une implication de ce médiateur dans la progression de ce désordre.

Le peptide défini selon l'invention est particulièrement bien adapté à une utilisation pour freiner la chute des cheveux en combattant une phase inflammatoire de l'alopécie.

Ainsi, l'invention a également pour objet l'utilisation dans une composition cosmétique ou pour la préparation d'un médicament, d'une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine, destinée à combattre une phase inflammatoire de l'alopécie.

Plus particulièrement, l'invention a pour objet l'utilisation dans une composition cosmétique ou pour la préparation d'un médicament, d'une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine, pour induire et/ou stimuler la croissance des cheveux et/ou freiner leur chute.

Le milieu physiologiquement acceptable dans lequel le peptide est utilisé selon l'invention peut être anhydre ou aqueux. On entend par milieu anhydre, un milieu solvant contenant moins de 1% d'eau. Ce milieu peut être constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine cosmétique ou pharmaceutique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaisse, de gel, d'émulsion, ou de crème. L'utilisation peut éventuellement se faire sous une forme pressurisée en aérosol ou vaporisée à partir d'un flacon pompe.

Il est possible aussi d'utiliser en association avec le peptide, des composés améliorant encore l'activité sur la repousse et/ou sur le freinage de la chute des cheveux, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les agents anti-inflammatoires stéroïdiens et non stéroïdiens, en particulier, l'hydrocortisone, ses sels et ses dérivés, l'acide niflumique, et autres ;
- les rétinoides, comme l'acide t-trans rétinoique, appelé encore Trétinoine, l'Isotrétinoine, le rétinol (ou vitamine A), et ses dérivés, tels que l'acétate, le palmitate ou le propionate, le Motrétinide, l'Etrétinate, le t-trans rétinoate de zinc ;
- les dérivés de pyrimidine, comme le 6-amino 1,2-dihydro 1-hydroxy 2-imino 4-pipéridino pyrimidine encore connu sous le nom de Minoxidil, et tels que décrits dans le brevet US 4 139 619 ;
- les agents antibactériens tels que les macrolides, les pyranosides et les tétracyclines, et notamment l'Erythromycine ;
- les agents antagonistes de calcium, comme la Cinnarizine et le Diltiazem ;
- des hormones, telles que l'estriol ou des analogues, ou la thyroxine et ses sels ;
- des agents antiandrogènes, tels que l'oxendolone, la spironolactone, le diéthylstilbestrol ;
- des inhibiteurs des 5-α-réductases ;
- des capteurs de radicaux OH, tels que le diméthylsulfoxyde.

A la liste ci-dessus, d'autres composés peuvent également être rajoutés, à savoir par exemple le Diazoxide, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'Anthraline, des caroténoides, les acides eicosatétraénoïque et eicosatriénoïque ou leurs esters et amides, la vitamine D et ses dérivés.

La composition cosmétique selon l'invention est à appliquer sur les zones alopéciques du cuir chevelu et des cheveux d'un individu, et est éventuellement laissée en contact plusieurs heures et est éventuellement à rincer. On peut, par exemple, appliquer la composition contenant une quantité efficace du peptide sur les cheveux et le cuir chevelu, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention à également pour objet un procédé de traitement cosmétique des cheveux et/ou du cuir chevelu destiné à induire et/ou stimuler la croissance des cheveux et/ou freiner leur chute, caractérisé par le fait qu'il consiste à appliquer sur les cheveux et/ou le cuir chevelu, une composition contenant une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine, à laisser celle-ci en contact avec les cheveux et/ou le cuir chevelu, et éventuellement à rincer.

Le procédé de traitement présente les caractéristiques d'un procédé cosmétique dans la mesure où il permet d'améliorer l'esthétique des cheveux en leur donnant une plus grande vigueur et un aspect amélioré.
On va maintenant donner à titre d'illustration des exemples qui ne sauraient limiter en aucune façon la portée de l'invention.
Dans les exemples qui suivent, Ac - LPV - NH₂ signifie Acétyl - Lys - Pro - Val - NH₂.

### Exemple 1 : Mesure de l'effet du tripeptide Ac-LPV-NH₂ et de l'hormone stimulatrice des mélanocytes de type α (α-MSH) sur l'allongement et la survie du follicule pileux maintenu in vitro.

Des follicules pileux viables in vitro sont préparés selon la technique décrite dans la demande de brevet n° 95-08465, déposée en France le 12 juillet 1995 par la demanderesse.

A partir d'une biopsie de scalp, une bande de cuir chevelu assez fine est isolée à l'aide d'un scalpel. Avec une micropince, le tissu adipeux entourant les follicules est éliminé en évitant d'endommager le bulbe pilaire. Sous un microscope, le follicule est coupé avec un scalpel pour le séparer de son environnement épidermique et dermique.

Les follicules sont ensuite mis en culture dans les puits de plaques 24 puits de type Costar, à raison d'un follicule par puits. Chaque puits contient 0,5 ml de milieu Williams E additionné de pénicilline et streptomycine à la concentration finale de 50 UI/ml, de glutamine à de 2 mM, d'Insuline de boeuf à 0,01 mg/ml, et d'hydrocortisone à 0,04 µg/ml.
Les follicules sont alors mesurés à l'aide d'un microscope muni d'un oculaire micrométrique.

Au bout de 24 heures les follicules sont de nouveau mesurés et ceux dont l'allongement est inférieur à 0,3 graduations du micromètre, correspondant à 0,16 mm, sont éliminés.
Les follicules conservés pour l'expérimentation sont alors mis en culture dans du milieu Williams E contenant ou ne contenant pas le tripeptide Ac-LPV-NH₂ ou l'α-MSH.

Les 5 conditions de culture suivantes ont été testées :
Condition A : milieu Williams E complet sans peptide.
Condition B : milieu Williams E complet + Ac-LPV-NH₂ à 10 µM
Condition C : milieu Williams E complet + Ac-LPV-NH₂ à 1 µM
Condition D : milieu Williams E complet + Ac-LPV-NH₂ à 0,1 µM
Condition E : milieu Williams E complet + α-MSH à 1 µM.

### Résultats :

| Condition A : | | | | |
|---|---|---|---|---|
| Jours | allongement moyen | écart type | n | % survie |
| 0 | 0,00 | 0,00 | 12 | 100 |
| 1 | 0,36 | 0,04 | 12 | 100 |
| 4 | 0,91 | 0,14 | 12 | 100 |
| 5 | 1,03 | 0,17 | 11 | 92 |
| 6 | 1,10 | 0,20 | 7 | 58 |
| 7 | 1,18 | 0,22 | 6 | 50 |
| 8 | 1,32 | 0,19 | 5 | 42 |
| 11 | 1,71 | 0,13 | 2 | 17 |
| 12 | 1,79 | 0,16 | 2 | 17 |

| Condition B : | | | | |
|---|---|---|---|---|
| Jours | allongement moyen | écart type | n | % survie |
| 0 | 0,00 | 0,00 | 12 | 100 |
| 1 | 0,38 | 0,06 | 12 | 100 |
| 4 | 1,11 | 0,15 | 12 | 100 |
| 5 | 1,33 | 0,18 | 12 | 100 |
| 6 | 1,43 | 0,20 | 12 | 100 |
| 7 | 1,54 | 0,24 | 12 | 100 |
| 8 | 1,74 | 0,27 | 11 | 92 |
| 11 | 1,96 | 0,41 | 10 | 83 |
| 12 | 2,40 | 0,40 | 5 | 42 |

| Condition C : | | | | |
|---|---|---|---|---|
| Jours | allongement moyen | écart type | n | % survie |
| 0 | 0,00 | 0,00 | 11 | 100 |
| 1 | 0,38 | 0,08 | 11 | 100 |
| 4 | 1,05 | 0,22 | 11 | 100 |
| 5 | 1,21 | 0,26 | 11 | 100 |
| 6 | 1,28 | 0,30 | 11 | 100 |
| 7 | 1,42 | 0,27 | 10 | 91 |
| 8 | 1,39 | 0,26 | 6 | 55 |
| 11 | 1,70 | 0,46 | 4 | 36 |

| Condition D : | | | | |
|---|---|---|---|---|
| Jours | allongement moyen | écart type | n | % survie |
| 0 | 0,00 | 0,00 | 12 | 100 |
| 1 | 0,37 | 0,05 | 12 | 100 |
| 4 | 1,10 | 0,14 | 12 | 100 |
| 5 | 1,22 | 0,17 | 12 | 100 |
| 6 | 1,28 | 0,18 | 12 | 100 |
| 7 | 1,42 | 0,22 | 10 | 83 |
| 8 | 1,58 | 0,28 | 6 | 50 |
| 11 | 2,08 | 0,26 | 5 | 42 |
| 12 | 2,11 | 0;00 | 2 | 17 |

| Condition E : | | | | |
|---|---|---|---|---|
| Jours | allongement moyen | écart type | n | % survie |
| 0 | 0,00 | 0,00 | 11 | 100 |
| 1 | 0,37 | 0,07 | 11 | 100 |
| 4 | 1,20 | 0,17 | 11 | 100 |
| 5 | 1,26 | 0,18 | 11 | 100 |
| 6 | 1,44 | 0,18 | 10 | 91 |
| 7 | 1,58 | 0,17 | 9 | 82 |
| 8 | 2,07 | 0,17 | 5 | 45 |
| 11 | 2,26 | 0,21 | 4 | 36 |

Ces résultats montrent que le tripeptide Ac-LPV-NH₂ et l'α-MSH favorisent l'allongement du follicule pileux en culture et augmentent son temps de survie. L'allongement du temps de survie correspond a un allongement de la phase du cycle dans laquelle se trouve le cheveu et permet ainsi d'en retarder la chute.

### Exemple 2 : Exemples de compositions contenant un peptide selon l'invention. Ces compositions sont obtenues par les techniques habituelles couramment utilisées en cosmétique ou en pharmacie.

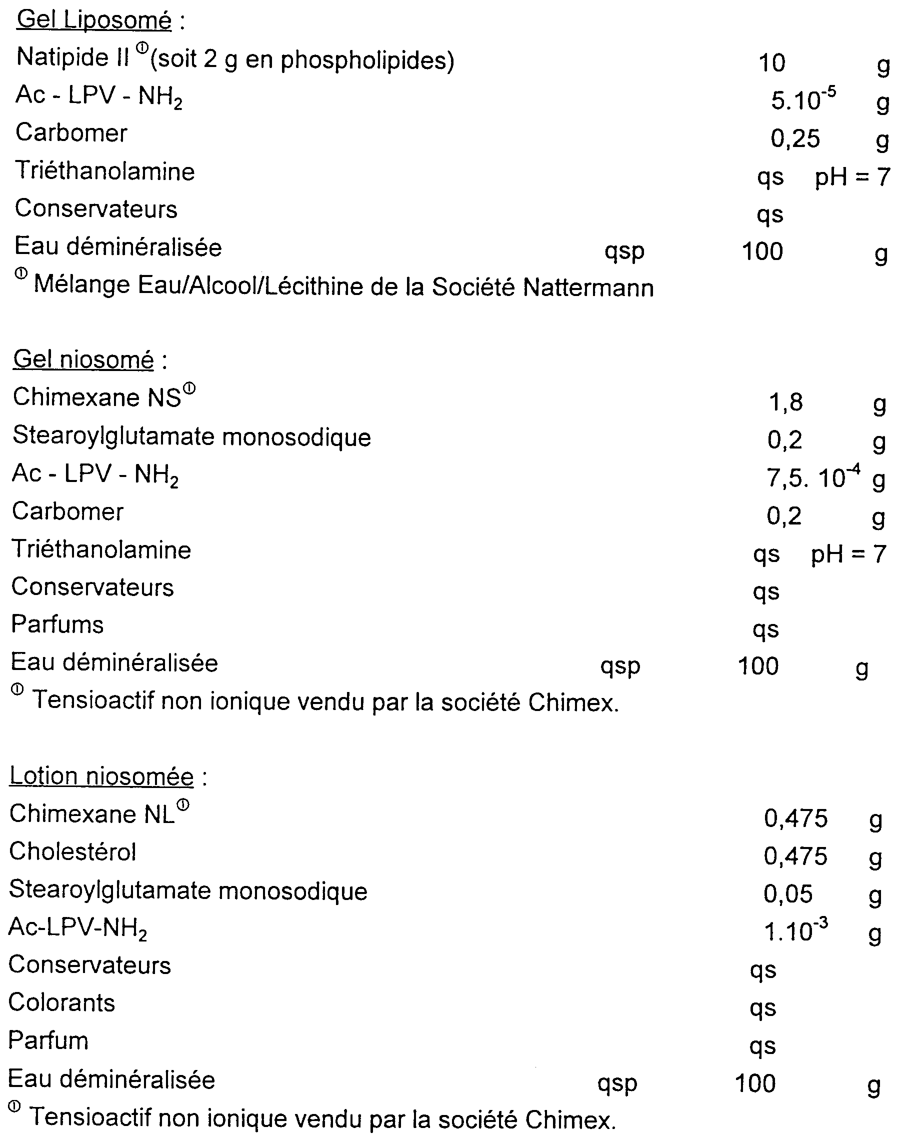

| Spray : | |
|---|---|
| Ac - LPV - NH₂ | 5.10⁻⁶ g |
| Minoxidil | 0,5 g |
| Ethanol à 95° | 55,1 g |
| Propylène glycol | 22,8 g |
| Parfum | qs |
| Eau déminéralisée | qsp 100 g |

| Crème de soin : émulsion H/E | |
|---|---|
| Alcool cétylstéarylique/Alcool cétylstéarylique oxyéthylénée à 33 moles d'oxyéthylène (80/20) | 5 g |
| Monostéarate de glycérol | 1,5 g |
| Alcool cétylique | 0,75 g |
| Huile de vaseline | 10 g |
| Polydimethylsiloxane | 0,75 g |
| Glycérine | 4 g |
| Conservateurs | qs |
| Ac-LPV-NH₂ | 5.10⁻³ g |
| Eau déminéralisée | qsp 100 g |

| Lotion quotidienne : | |
|---|---|
| Ac - LPV - NH₂ | 12,5. 10⁻⁶ g |
| 2,4 diaminopyrimidine 3-oxyde | 0,75 g |
| Ethanol à 95° | 30 g |
| Parfum | qs |
| Colorants | qs |
| Eau déminéralisée | qsp 100 g |

## Revendications

1. Utilisation dans une composition cosmétique ou pour la préparation d'un médicament, d'une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine, destinée à induire et/ou stimuler la croissance des cheveux et/ou freiner leur chute.

2. Utilisation selon la revendication précédente, caractérisée par le fait que le peptide est le tripeptide Lysine-Proline-Valine.

3. Utilisation selon l'une quelconque des revendications précédentes, caractérisée par le fait que le peptide est un peptide d'origine naturelle ou synthétique.

4. Utilisation selon l'une quelconque des revendications précédentes, caractérisée par le fait que les résidus acides aminés du tripeptide Lysine-Proline-Valine constituant le tripeptide sont sous la forme d'isomères optiques dextrogyre ou lévogyre.

5. Utilisation selon l'une quelconque des revendications précédentes, caractérisée par le fait que le peptide est sous une forme protégée ou non.

6. Utilisation selon la revendication précédente, caractérisée par le fait que la protection consiste en une protection basée soit sur l'acylation ou l'acétylation de l'extrémité amino-terminale, soit sur l'amidation de l'extrémité carboxy-terminale soit encore sur les deux.

7. Utilisation dans une composition cosmétique ou pour la préparation d'un médicament, d'une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine, destinée à combattre une phase inflammatoire de l'alopécie.

8. Utilisation selon la revendication précédente, pour induire et /ou stimuler la croissance des cheveux et/ou freiner leur chute.

9. Utilisation dans une composition cosmétique selon l'une quelconque des revendications précédentes, caractérisée par le fait que le tripeptide Lysine-Proline-Valine présent est utilisé à une concentration comprise entre 10⁻¹² M et 10⁻³ M et de préférence comprise entre 10⁻⁹ M et 10⁻⁴ M.

10. Utilisation pour la préparation d'un médicament selon l'une quelconque des revendications 1 à 6, caractérisée par le fait que le tripeptide Lysine-Proline-Valine est utilisé à une concentration comprise entre 10⁻¹² M et 1 M et de préférence comprise entre 10⁻⁶ M et 10⁻¹ M.

11. Procédé de traitement cosmétique des cheveux et/ou du cuir chevelu destiné à induire et/ou stimuler la croissance des cheveux et/ou freiner leur chute, caractérisé par le fait qu'il consiste à appliquer sur les cheveux et/ou le cuir chevelu, une composition contenant une quantité efficace d'au moins un peptide contenant le tripeptide Lysine-Proline-Valine ou de tout équivalent biologique fonctionnel, à l'exception de tout peptide ne contenant que la séquence Lysine-Proline-Valine immédiatement précédée d'une Histidine, à laisser celle-ci en contact avec les cheveux et/ou le cuir chevelu, et éventuellement à rincer.

## Claims

1. Use in a cosmetic composition or for the preparation of a medicament of an effective amount of at least one peptide containing the Lysine-Proline-Valine tripeptide or of any functional biological equivalent, with the exception of any peptide containing only the Lysine-Proline-Valine sequence immediately preceded by a Histidine, intended to induce and/or to stimulate hair growth and/or to slow down hair loss.

2. Use according to the preceding claim, characterized in that the peptide is the Lysine-Proline-Valine tripeptide.

3. Use according to either of the preceding claims, characterized in that the peptide is a peptide of natural or synthetic origin.

4. Use according to any one of the preceding claims, characterized in that the amino acids residues of the Lysine-Proline-Valine tripeptide constituting the tripeptide are in the form of dextrorotatory or laevorotatory optical isomers.

5. Use according to any one of the preceding claims, characterized in that the peptide is in a protected or non-protected form.

6. Use according to the preceding claim, characterized in that the protection consists of a protection based either on acylation or acetylation of the amino-terminal end or on amidation of the carboxy-terminal end or alternatively on both.

7. Use in a cosmetic composition or for the preparation of a medicament of an effective amount of at least one peptide containing the Lysine-Proline-Valine tripeptide or of any functional biological equivalent, with the exception of any peptide containing only the Lysine-Proline-Valine sequence immediately preceded by a Histidine, intended to combat an inflammatory stage of alopecia.

8. Use according to the preceding claim, for inducing and/or for stimulating hair growth and/or for slowing down hair loss.

9. Use in a cosmetic composition according to any one of the preceding claims, characterized in that the Lysine-Proline-Valine tripeptide present is used at a concentration of between 10⁻¹² M and 10⁻³ M and preferably of between 10⁻⁹ M and 10⁻⁴ M.

10. Use for the preparation of a medicament according to any one of Claims 1 to 6, characterized in that the Lysine-Proline-Valine tripeptide is used at a concentration of between 10⁻¹² M and 1 M and preferably of between 10⁻⁶ M and 10⁻¹ M.

11. Process for the cosmetic treatment of the hair and/or of the scalp intended to induce and/or to stimulate hair growth and/or to slow down hair loss, characterized in that it consists in applying, to the hair and/or the scalp, a composition containing an effective amount of at least one peptide containing the Lysine-Proline-Valine tripeptide or of any functional biological equivalent, with the exception of any peptide containing only the Lysine-Proline-Valine sequence immediately preceded by a Histidine, in leaving the composition in contact with the hair and/or the scalp, and optionally in rinsing.

## Patentansprüche

1. Verwendung einer wirksamen Menge mindestens eines Peptids, das die Tripeptidsequenz Lysin-Prolin-Valin aufweist, oder beliebiger in der biologischen Funktion äquivalenter Peptide mit Ausnahme aller Peptide, bei welchen die Sequenz Lysin-Prolin-Valin nur unmittelbar auf Histidin folgt, in einer kosmetischen Zusammensetzung oder zur Herstellung eines Arzneimittels zur Induzierung und/oder Stimulation des Haarwachstums und/oder zur Verlangsamung des Haarausfalls.

2. Verwendung nach dem vorhergehenden Anspruch, dadurch gekennzeichnet, daß das Peptid das Tripeptid Lysin-Prolin-Valin ist.

3. Verwendung nach einem der vorhergehenden Ansprüche, dadurch gekennzeichnet, daß das Peptid ein Peptid natürlichen oder synthetischen Ursprungs ist.

4. Verwendung nach einem der vorhergehenden Ansprüche, dadurch gekennzeichnet, daß die Aminosäuren des Tripeptids Lysin-Prolin-Valin, die das Tripeptid bilden, in Form der rechtsdrehenden oder linksdrehenden optischen Isomeren vorliegen.

5. Verwendung nach einem der vorhergehenden Ansprüche, dadurch gekennzeichnet, daß das Peptid in geschützter Form oder in nichtgeschützter Form vorliegt.

6. Verwendung nach dem vorhergehenden Anspruch, dadurch gekennzeichnet, daß der Schutz auf der Acylierung oder Acetylierung der Amino-Endgruppe oder auf der Amidbildung an der Carboxy-Endgruppe oder auf beiden Maßnahmen beruht.

7. Verwendung einer wirksamen Menge mindestens eines Peptids, das die Tripeptidsequenz Lysin-Prolin-Valin aufweist, oder beliebiger in der biologischen Funktion äquivalenter Peptide mit Ausnahme aller Peptide, bei welchen die Sequenz Lysin-Prolin-Valin nur unmittelbar auf Histidin folgt, in einer kosmetischen Zusammensetzung oder zur Herstellung eines Arzneimittels zur Bekämpfung einer entzündlichen Phase der Alopezie.

8. Verwendung nach dem vorhergehenden Anspruch zur Induzierung und/oder zur Stimulation des Haarwachstums und/oder zur Verlangsamung des Haarausfalls.

9. Verwendung in einer kosmetischen Zusammensetzung nach einem der vorhergehenden Ansprüche, dadurch gekennzeichnet, daß das eingesetzte Tripeptid Lysin-Prolin-Valin in einer Konzentration im Bereich von 10⁻¹² bis 10⁻³ M und vorzugsweise im Bereich von 10⁻⁹ und 10⁻⁴ M verwendet wird.

10. Verwendung zur Herstellung eines Arzneimittels nach einem der Ansprüche 1 bis 6, dadurch gekennzeichnet, daß das Tripeptid Lysin-Prolin-Valin in einer Konzentration im Bereich von 10⁻¹² bis 1 M und vorzugsweise von 10⁻⁶ bis 10⁻¹ M verwendet wird.

11. Verfahren zur kosmetischen Behandlung der Haare und/oder der Kopfhaut zur Induzierung und/oder Stimulation des Haarwachstums und/oder zur Verlangsamung des Haarausfalls, dadurch gekennzeichnet, daß es darin besteht, auf das Haar und/oder die Kopfhaut eine Zusammensetzung aufzutragen, die eine wirksame Menge mindestens eines Peptids, das die Tripeptidsequenz Lysin-Prolin-Valin aufweist, oder beliebiger in der biologischen Funktion äquivalenter Peptide enthält, mit Ausnahme aller Peptide, bei denen die Sequenz Lysin-Prolin-Valin ausschließlich unmittelbar auf Histidin folgt, das Haar und/oder die Kopfhaut mit der Zusammensetzung in Kontakt zu lassen und ggf. zu spülen.
